# EUROPEAN PATENT SPECIFICATION

(11) **EP 2 238 976 B1**
(45) Date of publication and mention of the grant of the patent: **27.06.2012**
(21) Application number: 09157337.8
(22) Date of filing: 03.04.2009
(51) Int. Cl.: A61K 9/70, A61K 31/496, A61P 25/18

(54) **Oral films comprising 7-[4-[4-(2,3-dichlorophenyl)piperazin-1-yl]butoxy]-3,4-dihydro- 1H-quinolin-2-one base or salts or hydrates thereof**
Oraler Film mit 7-[4-[4-(2,3-dichlorophenyl)piperazin-1-yl]butoxy]-3,4-dihydro-1H-chinolin-2-on-Base oder Salze oder Hydrate davon
Films oraux comportant une base 7-[4-[4-(2,3-dichlorophenyl)piperazin-1-yl]butoxy]-3,4-dihydro- 1H-quinolin-2-one ou sels ou hydrates associés

(43) Date of publication of application: 13.10.2010
(73) Proprietor: Hexal AG, 83607 Holzkirchen (DE)
(72) Inventor: Krekeler, Andreas, 83607 Holzkirchen (DE); Buggele, Nicole, 83607 Holzkirchen (DE); Born, Max, 83607 Holzkirchen (DE); Grundsteiner, Sigrid, 83607 Holzkirchen (DE)
(74) Representative: Ellis, Robin Patrick

(56) References cited:
- EP-A- 1 752 127
- WO-A-2006/079548

## Description

### Field of the invention:

The present invention relates to oral films comprising the active pharmaceutical ingredient (API) 7-[4-[4-(2,3-dichlorophenyl)piperazin-1-yl]butoxy]-3,4-dihydro-1H-quinolin-2-one and methods for producing oral films comprising 7-[4-[4-(2,3-dichlorophenyl)piperazin-1-yl]butoxy]-3,4-dihydro-1H-quinolin-2-one.

### Background of the invention:

7-[4-[4-(2,3-dichlorophenyl)piperazin-1-yl]butoxy]-3,4-dihydro-1H-quinolin-2-one (referred to herein as aripiprazole) is a carbostyril derivative that was first claimed in EP0367141. In the same publication, aripiprazole was identified as being useful in the treatment of schizophrenia.

Following aripiprazole's publication in the early 1990s there was a huge number of patent applications and scientific papers covering various polymorphs, salts and solvates of aripiprazole.

The Proceedings of the 4th Japanese-Korean Symposium on Separation Technology (October 6-8, 1996) disclosed both hydrous and anhydrous forms of aripiprazole which were referred to as Types 1, 2 and 3.

Subsequent patent applications claimed a variety of aripiprazole forms including WO 03/026659 (Forms A to G and amorphous aripiprazole); EP1618103 (Forms II, III, IV); WO 05/058835 (Forms I, II, VI, VIII, X to XII, XIV, XIX and XX); WO 06/012237 (Form Alpha); WO 06/053780 (methanolate & ethanolate); WO 06/079548 (Form X, hemiethanolate & methanol solvate) and WO 07/004061 (Forms H to L). Although a number of the polymorphs and solvates disclosed in these applications overlap, the quantity of patent applications concerning this API highlight the level of activity that has surrounded this API.

Patent application EP 1 752 127 A discloses edible film preparations comprising aripiprazole and hydroxypropylcellulose.

Since 2004, aripiprazole has been marketed by BMS & Otsuka under the brand name Abilify® as film coated and orally disintegrating tablets, and as an injectable solution, for the treatment of schizophrenia, manic or mixed episodes, bipolar disorder and major depressive disorder.

In recent years there has been interest in developing oral films (ORFs) as a delivery medium for APIs. ORFs have been known since the late 1990s, when they were first introduced as consumer products such as Listerine POCKETPAKS^{™} and they are already used as a pharmaceutical delivery medium in products such as Theraflu^{®} Thin Strips, Gas-X^{®} Thin Strips, Triaminic Thin Strips^{®}, Children's Benadryl^{®}, Chloraseptic^{®}. Oral films are intended for application in the oral cavity where they quickly dissolve prior to swallowing.

More recently, ORFs have been recognised as a particularly useful delivery medium with difficult patient groups who are reluctant and/or resistant to taking medication. API administration using ORFs has been seen to result in a much higher compliance rate due to the fact that they are not easily spat out after administration and they provide alleviated administration for patients who have difficulty swallowing bulky tablets.

However, despite their advantages over the more traditional pharmaceutical formulations, such as film coated tablets and orally disintegrating tablets, ORFs are very difficult to manufacture and are often not a suitable delivery medium for many APIs due to the unfavourable conditions the API has to be subjected to during the manufacturing process.

Problems associated with the manufacture of ORFs include:
1. Stability of the coating mass (either solution, emulsion or dispersion) which has to remain homogenous during the whole production process;
2. Properties of the resulting laminate and film must be sufficient to enable punching and safe removal of film from the packaging but also be capable of rapidly disintegrating in the oral cavity and provide rapid release of the dosage form (e.g. suitable tensile strength must be achieved without compromising the rate of dissolution);
3. Physical and chemical stability of the API is difficult to achieve because impurities might increase due to the aqueous environment and higher temperatures are required during the manufacturing process which tend to alter the desired polymorphic form; this in turn can affect the final release properties of the API.

The incorporation of aripiprazole into an oral film presents particular problems because its low solubility in water makes making an oral film out of a solution very difficult When completely dissolved in certain solvents it has been found that aripiprazole will crystallize out into less soluble solvate forms which are not suitable for achieving the necessary API release rate, following oral administration, *in vivo.*

The problem of bioavailability also highlights the importance of finding a polymorph or solvate of aripiprazole that can not only withstand the rigorous manufacturing process required for oral films but also provide the necessary stability and release properties (cₘₐₓ, tₘₐₓ and AUC) in the finished oral film.

### Summary of Invention:

According to the present invention the aforementioned problems have been solved by providing an oral film comprising 7-[4-[4-(2,3-dichlorophenyl)piperazin-1-yl]butoxy]-3,4-dihydro-1H-quinolin-2-one base or salts or hydrates thereof, characterised in that the 7-[4-[4-(2,3-dichlorophenyl)piperazin-1-yl]butoxyl-3,4-dlhydro-1H-quinolin-2-one base or salt or hydrate thereof has a X-ray powder diffraction pattern having peaks at 10.0, 11.6, 15.7, 16.3, 18.5, 20.4, 21.8, 22.2 and 23.3 degrees two-theta, ± 0.2 degrees two-theta.

In an additional aspect of the present invention the oral films comprise 10 to 40wt% 7-[4-[4-(2,3-dichlorophenyl) piperazin-1-yl]butoxyl-3,4-dihydro-1H-quinolin-2-one and may further comprise a film forming polymer selected from the group consisting of polysaccharides, polypeptides, synthetic polymers or mixtures thereof.

Preferably, the oral film according to the present invention comprises: 15-40wt% aripiprazole; 5-30wt% film forming polymer; 0-5wt% surfactant; 10-40wt% filler; 5-50wt% plasticizer; and 0-5wt% flavouring agent.

The present invention also relates to a method of manufacturing an oral film, comprising 7-[4-[4-(2,3-dichlorophenyl)piperazin-1-yl]butoxy]-3,4-dihydro-1H-quinolin-2-one base or salts or hydrates thereof comprising the steps of:
(a) adding 7-[4-[4-(2,3-dichlorophenyl)piperazin-1-yl]butoxy]-3,4-dihydro-1H-quinolin-2-one to a solvent together with at least one excipient selected from film forming polymers, fillers, plasticizers and flavouring agents; and
(b) drying the mixture to form the oral film

In particular, the method of the invention uses a solvent selected from at least one of 2-propanol and water. In a further embodiment of the invention the solvent is a mixture of 2-propanol and water wherein it is preferred that the ratio of 2-propanol to water is from 1:3 to 1:1.

### Figures:

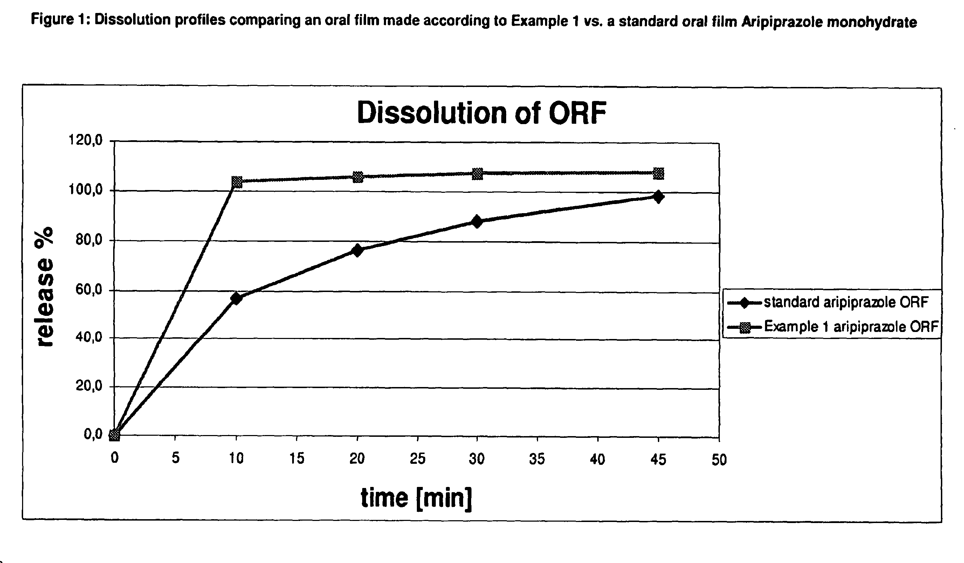

Fig. 1 shows a comparison of dissolution profiles of an oral film made according to Example 1 vs. a standard oral film containing aripiprazole monohydrate. The dissolution was performed using Apparatus 2, PhEur., in 1000 ml medium of pH 4.0, at 75 rpm and 37°C.

### Description:

In addition to aripiprazole, the oral films of the present invention comprise film forming polymers and preferably comprise further excipients including surfactants; fillers; plasticizers and flavourings which are discussed in further detail hereafter.

Preferably the film forming polymers are selected from, but are not limited to, polysaccharides (e.g. cellulose and derivates thereof, starch and derivates thereof, carageen), synthetic polymers (e.g. polyvinylalcohol, polyvinylpyrrolidone, acrylates), polypeptides (collagen, gelatine) and mixtures thereof. Preferred cellulose derivatives include hydroxypropyl methyl cellulose, in particular those with an apparent viscosity of between 30-70 centipoises, and ideally 40-60 centipoises. Apparent viscosity is measured as described in the USP monograph for Hypromellose. Briefly, an aqueous 2 % m/m solution of hydroxypropyl methyl cellulose is analyzed with a Ubbelohde type viscosimeter at 20° C.

Preferably, the percentage of film forming polymer in the finished ORF is between 0-40wt%, preferably 5-30wt% and most preferably 10-20wt% of the total weight of the finished oral film. Suitable surfactants include, but are not limited to, Tween 80, isopropyl palmitate and dibutyl sebacate. The amount of surfactant present in the finished ORF can be between 0-5wt%, preferably 0,01-3wt% and most preferably 0,1-1wt% of the total weight of the finished oral film.

Fillers may be selected from, but are not limited to, TiO₂, SiO₂, microcrystalline cellulose, maltodextrin and can be present in an amount from 0-50wt%, preferably 10-40wt% and most preferably 20-30wt% of the total weight of the finished oral film.

Plasticizers such as glycerol and propylene glycol may be used in quantities ranging from 5-50wt%, preferably 10-40wt% and most preferably 20-30wt% and flavouring agents such as sucralose and ethylvanillin may also be included in low quantities of between 0-5wt%, preferably 0,01-3wt% and most preferably 0,1-1wt% of the total weight of the finished oral film.

To produce the oral films of the present invention the aripiprazole and the aforementioned excipients are initially added to a solvent.

The solvent is preferably selected from water, ethanol, 2-propanol, 1- propanol, acetone, methylethyl ketone, tetrahydrofuran, methanol, ethyl acetate, ethyl ether, acetic acid, formic acid, acetonitrile, dimethyl sulfoxide, ethyl formate, heptane, anisole, 1-butanol, 2-butanol, butyl acetate, tert-butylmethylether, cumene, isobutyl acetate, isopropyl acetate, methyl acetate, 3-methyl-1-butanol, methylethyl ketone, methylisobutyl ketone, 2-methyl-1-propanol, pentane, 1-pentanol, propyl acetate, chlorobenzene, chloroform, cyclehexane, 1,2-dichloroehtylene, dichloromethane, 1,2.dimethoxyethane, N,N-dimethylacetamide, N,N-dimethylformamide, 1,4-dioxane, 2-ethoxyethanol, ethyleneglycol, formamide, hexane, N-methylpyrrolidone, nitromethane, pyridine, sulfolane, tetraline, toluene, 1,1,1-trichloroethane, 1,1,2-trichloroethene, xylene, and mixtures thereof. Water, ethanol, 2-propanol, 1-propanol and acetone or mixtures thereof are preferred. When two solvents are used they may be combined in various ratios in the range including 1:9; 1:4; 3:7; 2:3; 2:1 and preferably 1:1. depending on the solvents used. Most preferably a water:2-propanol ratio of 1:1 is used, although ratios of 2:1; 3:2; 3:1 and higher are also suitable.

Addition of the excipients to the solvent may be performed between 5-40°C, preferably between 10-35°C, more preferably between 15-30°C and ideally at 20-25°C. Stirring of the mixture during addition is recommended but not essential. If used, stirring preferably lasts for 20 to 300 minutes, more preferably 40 to 200 minutes and most preferably 50 to 120 minutes.

Although the order in which the aripiprazole and excipients are added may vary depending on the solvent and excipients, it is preferred that the surfactant is first dissolved in the solvent prior to the addition of the other excipients and aripiprazole. Furthermore, it may be preferable to only add the aripiprazole after the addition of all other excipients.

The finished ORF may comprise between 10-50wt% aripiprazole base or salts or hydrates thereof; preferably it contains between 15-40wt% and most preferably between 20-35wt% of the total weight of the finished oral film.

Any form of aripiprazole may be used in the oral films of the present invention but the preferred polymorphic form is that referred to herein as Form X as shown in Table 1. Most preferably, Form X is obtained according to Example 13 of WO2006/079548.

**Table 1:**

| Form X | |
|---|---|
| 2-θ Value | Intensity |
| 5,3 | 7 |
| 10,0 | 22 |
| 10,7 | 7 |
| 11,1 | 8 |
| 11,6 | 22 |
| 12,6 | 10 |
| 15,7 | 23 |
| 16,3 | 41 |
| 18,5 | 21 |
| 18,9 | 8 |
| 19,8 | 14 |
| 20,0 | 12 |
| 20,4 | 20 |
| 21,8 | 39 |
| 22,2 | 100 |
| 23,3 | 24 |
| 24,4 | 9 |
| 26,0 | 13 |
| 27,0 | 9 |
| 28,8 | 13 |
| 33,6 | 6 |
| 35,3 | 7 |
| 35,6 | 6 |
| 39,4 | 6 |

Following addition of all of the excipients and the aripiprazole, the mixture is left to homogenise. Preferably homogenisation occurs in less than one hour but, depending on the solvent and excipients used, homogenisation may take between 1-24 hours; 3-20 hours; and most likely 6-16 hours or overnight (12 hours).

Finally, the homogenised mixture is spread on a support foil and standard oral film producing equipment (e.g. a spreading knife) is used to achieve the desired thickness of film. The film is then dried at between 40 to 110°C; preferably 40 to 80 °C and most preferably 50 °C for between 20-100 minutes; preferably 30-60 minutes and most preferably for 45 minutes.

Once dried the resulting film is cut and packaged using standard oral film technology and techniques.

Use of the terms "mixing" or "mixture" in this specification is intended to include solutions; emulsions; dispersions and suspensions and the formation thereof.

The present invention is now described in more detail by, but is not limited to, the following Examples.

### Example 1:

The coating mass was prepared at 20°C by combining 24.38g water with 13.13g 2-propanol and thoroughly admixing 37mg Tween 80 for 5 minutes. 3.75g aripiprazole anhydrate (Form X) was added and stirred for another 5 minutes at 200 rotations per minute (rpm). 3g Glycerol and 1,83g of Maltodextrin (dextrose equivalent 13.0-17.0) were added and stirred for another 2 minutes. Whilst stirring, 1.583g Emcocel SP15 and 1.800g Metolose 60 SH-50 was slowly added and stirring of the coating mass was continued for 2 hours.

The coating mass was cast on a siliconized PET foil so that after drying a coat weight of 80g/m² was obtained. The resulting laminate was dried at 50 °C for 45 minutes. After drying, the laminate was cut into the desired shape, the foil was removed and the film packaged.

### Example 2:

A first mixture was prepared at 20°C by mixing 37mg Tween 80 in 24.38g water and adding 3g Glycerol and 1.83 g Maltodextrin (dextrose equivalent 13.0-17.0). Whilst stirring, 1.583g Emcocel SP15 and 1.800g Metolose 60 SH-50 was slowly added and stirring of the coating mass was continued for 1 hour.

A second mixture was prepared by suspending 3.75g aripiprazole anhydrate (Form X) in 13.13g 2-propanol and stirring for 5 minutes.

The second mixture was then added to the first mixture whilst stirring and stirring was continued for 1 hour.

The coating mass was cast on a siliconised PET foil so that after drying a coat weight of 80g/m² was obtained. The resulting laminate was dried at 50 °C for 45 minutes. After drying, the laminate was cut into the desired shape, the foil was removed and the film packaged.

### Dissolution Results:

The dissolution rate of the oral film made according to Example 1 was compared to the dissolution rate of a standard oral film comprising aripiprazole monohydrate. The dissolution was performed using Apparatus 2, PhEur., at 75 rpm in a 1000 ml medium at pH 4.0 and 37°C using 6 cm² samples of the ORF. The results, shown in Table 2 and Figure 1, show that the oral films of the present invention have a faster release profile when compared to standard oral film comprising aripiprazole monohydrate.

**Table 2:**

| **Minutes** | **Monohydrate wt% release** | | | | **Anhydrate wt% release** | | |
|---|---|---|---|---|---|---|---|
| | **Trial No.** | | | | **Trial No.** | | |
| | 1 | 2 | 3 | | 1 | 2 | 3 |
| 0 | 0 | 0 | 0 | | 0 | 0 | 0 |
| 10 | 58 | 56,4 | 55,4 | | 103 | 105 | 103,6 |
| 20 | 77,4 | 75,7 | 76 | | 104,6 | 107,5 | 105,4 |
| 30 | 89,7 | 87,4 | 87,9 | | 106,8 | 107,7 | 106,9 |
| 45 | 99,1 | 97,3 | 97,8 | | 108 | 108,3 | 107,2 |

## Claims

1. An oral film comprising 7-[4-[4-(2,3-dichlorophenyl)piperazin-1-yl]butoxy]-3,4-dihydro-1H-quinolin-2-one base or salts or hydrates thereof, **characterised in that** the 7-[4-[4-(2,3-dichlorophenyl)piperazin-1-yl]butoxy]-3,4-dihydro-1H-quinolin-2-one base or salt or hydrate thereof has a X-ray powder diffraction pattern having peaks at 10.0, 11.6, 15.7, 16.3, 18.5, 20.4, 21.8, 22.2 and 23.3 degrees two-theta, ± 0.2 degrees two-theta.

2. The oral film according to any preceding claim, comprising 10wt% to 50wt% 7-[4-[4-(2,3-dichlorophenyl)piperazin-1-yl]butoxy]-3,4-dihydro-1H-quinolin-2-one of the finished oral film.

3. The oral film according to any preceding claim, comprising a film forming polymer selected from the group of polysaccharides, polypeptides, synthetic polymers or mixtures thereof.

4. The oral film according to any preceding claim comprising: 15-40wt% aripiprazole; 5-30wt% film forming polymer; 0-5wt% surfactant; 10-40wt% filler; 5-50wt% plasticizer; and 0-5wt% flavouring agent of the finished oral film.

5. A method of manufacturing the oral film according to any proceeding claim, comprising the steps of:
(a) adding 7-[4-[4-(2,3-dichlorophenyl)piperazin-1-yl]butoxy]-3,4-dihydro-1H-quinolin-2-one to a solvent together with at least one excipient selected from film forming polymers, fillers, plasticizers and flavouring agents; and
(b) drying the mixture to form the oral film

6. The method according to claim 6 wherein the solvent is selected from water, ethanol, 2-propanol, 1- propanol, acetone, methylethylketone, tetrahydrofuran, methanol, ethyl acetate, ethyl ether, acetic acid, formic acid, acetonitrile, dimethyl sulfoxide, ethyl formate, heptane, anisole, 1-butanol, 2-butanol, butyl acetate, tert-butylmethylether, cumene, isobutyl acetate, isopropyl acetate, methyl acetate, 3-methyl-1-butanol, methylisobutyl ketone, 2-methyl-1-propanol, pentane, 1-pentanol, propyl acetate, chlorobenzene, chloroform, cyclohexane, 1,2-dichloroehtylene, dichloromethane, 1,2.dimethoxyethane, N,N-dimethylacetamide, N,N-dimethylformamide, 1,4-dioxane, 2-ethoxyethanol, ethyleneglycol, formamide, hexane, N-methylpyrrolidone, nitromethane, pyridine, sulfolane, tetraline, toluene, 1,1,1-trichloroethane, 1,1,2-trichloroethene, xylene, and mixtures thereof.

7. The method according to claim 7 wherein the solvent is selected from at least one of 2-propanol and water.

8. The method according to claims 6 to 8 wherein the solvent is a mixture of 2-propanol and water.

9. The method according to claims 6 to 9 wherein the ratio of 2-propanol to water is from 1:3 to 1:1.

## Patentansprüche

1. Ein oraler Filmstreifen, welcher 7-[4-[4-(2,3-dichlorophenyl)piperazin-1-yl]butoxy]-3,4-dihydro-1H-chinolin-2-one-Basen oder Salze oder Hydrate davon enthält, **gekennzeichnet dadurch, dass** die 7-[4-[4-(2,3-dichlorophenyl)piperazin-1-yl]butoxy]-3,4-dihydro-1H-chinolin-2-one-Basen oder Salze oder Hydrate davon eine Röntgenstrahlpulverdiffraktion mit Spitzen bei 10,0, 11,6, 15,7, 16,3, 18,5, 20,4, 21,8, 22,2 und 23,3 Grad Zwei-Theta ± 0,2 Grad Zwei-Theta aufweisen.

2. Der orale Film enthält gemäß jeglichen weitergehenden Ansprüchen 10 bis 50 Gewichtsprozent 7-[4-[4-(2,3-dichlorophenyl)piperazin-1-yl]butoxy]-3,4-dihydro-1H-chinolin-2-one des fertigen oralen Films.

3. Der orale Film enthält gemäß jeglichen weitergehenden Ansprüchen ein filmbildendes Polymer, ausgewählt aus der Gruppe der Polysaccharide, Polypeptide, synthetischen Polymere oder Mischungen davon.

4. Der orale Film enthält gemäß jeglichen weitergehenden Ansprüchen: 15-40 Gewichtsprozent Aripiprazol; 5-30 Gewichtsprozent filmbildendes Polymer; 0-5 Gewichtsprozent oberflächenaktive Substanz; 10-40 Gewichtsprozent Füllstoff; 5-50 Gewichtsprozent Weichmacher; und 0-5 Gewichtsprozent Aromastoff, jeweils vom fertigen oralen Film.

5. Die Herstellungsmethode des oralen Films umfasst gemäß jeglichen weitergehenden Ansprüchen folgende Schritte:
(a) Zugabe von 7-[4-[4-(2,3-dichlorophenyl)piperazin-1-yl]butoxy]-3,4-dihydro-1H-quinolin-2-one zu einer Lösung zusammen mit mindestens einem Trägerstoff, ausgewählt aus den filmbildenden Polymeren, Füllstoffen, Weichmachern und Aromastoffen; sowie
(b) Trocknung der Mischung zur Bildung des oralen Films

6. Die Methode gemäß Anspruch 6, wobei die Lösung aus Wasser, Ethanol, 2-Propanol, 1- Propanol, Aceton, Methylethylketon, Tetrahydrofuran, Methanol, Ethylacetat, Ethylether, Ethansäure, Methansäure, Acetonitril, Dimethylsulfoxid, Ethylformiat, Heptan, Anisol, 1-Butanol, 2-Butanol, Butylacetat, Tertbutylmethylether, Cumen, Isobutylacetat, Isopropylacetat, Methylacetat, 3-Methyl-1-Butanol, Methylisobutylketon, 2-Methyl-1-Propanol, Pentan, 1-Pentanol, Propylacetat, Chlorobenzen, Chloroform, Cyclohexan, 1,2-Dichloroehtylen, Dichloromethan, 1,2.Dimethoxyethan, N,N-Dimethylacetamid, N,N-Dimethylformamid, 1,4-Dioxan, 2-Ethoxyethanol, Ethyleneglycol, Formamid, Hexan, N-Methylpyrrolidon, Nitromethan, Pyridin, Sulfolan, Tetralin, Toluol, 1,1,1-Trichloroethan, 1,1,2-Trichloroethen, Xylen und Mischungen davon besteht.

7. Die Methode gemäß Anspruch 7, wobei die Lösung aus mindestens einem der Stoffe 2-Propanol und Wasser besteht.

8. Die Methode gemäß den Ansprüchen 6 bis 8, wobei die Lösung eine Mischung aus 2-Propanol und Wasser ist.

9. Die Methode gemäß den Ansprüchen 6 bis 9, wobei das Mischungsverhältnis von 2-Propanol und Wasser zwischen 1:3 und 1:1 liegt.

## Revendications

1. Un film oral comprenant une base 7-[4-[4-(2,3-dichlorophényl)pipérazin-1-yl]butoxy]-3,4-dihydro-1H-quinolin-2-one ou des sels ou hydrates de cette substance, **caractérisé en ce que** la base 7-[4-[4-(2,3-dichlorophényl)pipérazin-1-yl]butoxy]-3,4-dihydro-1H-quinolin-2-one ou le sel ou l'hydrate de cette substance a un profil de diffraction de rayons X sur poudre avec des pics à 10.0, 11.6, 15.7, 16.3, 18.5, 20.4, 21.8, 22.2 et 23.3 degrés deux-thêta, ± 0.2 degré deux-thêta.

2. Le film oral selon la revendication précédente, avec 10wt% à 50wt% de 7-[4-[4-(2,3-dichlorophényl)pipérazin-1-yl]butoxy]-3,4-dihydro-1H-quinolin-2-one dans le film oral fini.

3. Le film oral selon une des revendications précédentes comprenant un polymère filmogène sélectionné dans le groupe des polysaccharides, polypeptides, polymères synthétiques ou des mélanges de ces éléments.

4. Le film oral selon une des revendications précédentes comprenant : 15-40wt% d'aripiprazole, 5-30wt% de polymère filmogène, 0-5wt% de surfactant, 10-40wt% de matière de charge, 5-50wt% de plastifiant et 0-5wt% de substance aromatisante dans le film oral fini.

5. Une méthode de fabrication du film oral selon une des revendications précédentes comprenant les étapes suivantes :
(a) ajouter 7-[4-[4-(2,3-dichlorophényl)pipérazin-1-yl]butoxy]-3,4-dihydro-1H-quinolin-2-one à un solvant avec au moins un excipient parmi les éléments suivants : polymères filmogènes, matières de charge, plastifiants et substances aromatisantes ; et
(b) sécher la préparation afin de former le film oral

6. La méthode selon la revendication 6, où le solvant est un des éléments suivants : eau, éthanol, 2-propanol, 1-propanol, acétone, méthyléthylcétone, tétrahydrofurane, méthanol, acétate d'éthyle, éther éthylique, acide acétique, acide formique, acétonitrile, diméthylsulfoxyde, formiate d'éthyle, heptane, anisole, 1-butanol, 2-butanol, acétate de butyle, tert-butylméthyléther, cumène, acétate d'isobutyle, acétate d'isopropyle, acétate de méthyle, 3-méthyl-1-butanol, méthylisobutylcétone, 2-méthyl-1-propanol, pentane, 1-pentanol, acétate de propyle, chlorobenzène, chloroforme, cyclohexane, 1,2-dichloroéthylène, dichlorométhane, 1,2-diméthoxyéthane, N,N-diméthylacétamide, N,N-diméthylformamide, 1,4-dioxane, 2-ethoxyéthanol, éthylène glycol, formamide, hexane, N-méthypyrrolidone, nitrométhane, pyridine, sulfolane, tétraline, toluène, 1,1,1-trichloroéthane, 1,1,2-trichloroéthène, xylène, et mélanges de ces substances.

7. La méthode selon la revendication 7, où le solvant est au moins un des éléments suivants : 2-propanol, eau.

8. La méthode selon les revendications 6 à 8, où le solvant est un mélange de 2-propanol et d'eau.

9. La méthode selon les revendications 6 à 9, où la proportion de 2-propanol et d'eau est entre 1:3 et 1:1.
